Europäisches Patentamt

European Patent Office

Office européen des brevets

(11) Numéro de publication : **0 330 583 B1**

(12) # FASCICULE DE BREVET EUROPEEN

(45) Date de publication du fascicule du brevet :
**18.08.93 Bulletin 93/33**

(51) Int. Cl.⁵ : **A61K 7/48,** A61K 31/68,
A61K 31/07

(21) Numéro de dépôt : **89400531.3**

(22) Date de dépôt : **24.02.89**

(54) **Méthode pour améliorer l'aspect esthétique de la peau à l'aide de mélanges polyvitaminiques et compositions cosmétiques pour sa mise en oeuvre.**

Demande divisionnaire 92119446.0 déposée le 24/02/89.

(30) Priorité : **26.02.88 LU 87145**

(43) Date de publication de la demande :
**30.08.89 Bulletin 89/35**

(45) Mention de la délivrance du brevet :
**18.08.93 Bulletin 93/33**

(84) Etats contractants désignés :
**AT BE CH DE ES FR GB GR IT LI NL SE**

(56) Documents cités :
**WO-A-82/02833
WO-A-86/01402
GB-A- 1 431 841
GB-A- 2 212 722
CHEMICAL ABSTRACTS, vol. 73, no. 10, 7 septembre 1970, page 211, résumé no. 48487y, Columbus, Ohio, US; B. ELLOT: "Vitamins [in] cosmetics", & KOSMET.-PARFUM-DROGEN RUNDSCH. 1970, 17(3-4), 33-4,36-8**

(56) Documents cités :
**STN FILE SERVER (KARLSRUHE), CHEMICAL ABSTRACTS, vol. 77, no. 24, 1972, résumé no. 156274t, Columbus, Ohio, US; E. SHERR-BRUZER: "Additives which enhance the efficiency of cosmetics", & OLAJ, SZAPPAN, KOZMET., 21(1), 18-21**

(73) Titulaire : **L'OREAL
14, Rue Royale
F-75008 Paris (FR)**

(72) Inventeur : **Griat, Jacqueline
11, Quai de la Baronnie
F-94480 Ablon (FR)**
Inventeur : **Soudant, Etienne
23, rue Mozart
F-92160 Antony (FR)**
Inventeur : **Zabotto, Arlette
24, rue Sarrette
F-75014 Paris (FR)**
Inventeur : **Fanchon, Chantal
105, Quai Branly
F-75015 Paris (FR)**
Inventeur : **Pradier, François
52, Rue André Salel
F-92260 Fontenay aux Roses (FR)**

(74) Mandataire : **Stalla-Bourdillon, Bernard
CABINET NONY & CIE 29, rue Cambacérès
F-75008 Paris (FR)**

## Description

La présente invention a pour objet une méthode pour améliorer l'aspect esthétique de la peau, notamment du visage, à l'aide d'associations polyvitaminiques ainsi qu'une composition cosmétique pour sa mise en oeuvre.

Il est connu que certaines associations équilibrées de vitamines permettent de traiter les sujets présentant une déficience de l'état général due à des fatigues physiques ou intellectuelles, au surmenage ou encore à une alimentation déséquilibrée. Par ailleurs, de tels traitements à base de vitamines sont particulièrement recommandés en période de croissance ou de grossesse et en convalescence.

Ces mélanges de vitamines qui peuvent également être associés à des sels minéraux et des oligoéléments contiennent, à des doses thérapeutiquement actives, essentiellement les vitamines suivantes: A, D2, B1, B2, B5, B6, C et E ainsi que de l'acide nicotinique (vitamine PP) et de la biotine (vitamine H).

Il est également connu, à la suite de nombreuses études scientifiques, que la peau est un tissu particulièrement sensible aux carences nutritionnelles en vitamines. La carence en vitamines du groupe B, notamment en vitamines B1, B2 et B6 ainsi que la carence en vitamines C et A engendrent des désordres cutanés qui se manifestent essentiellement par un aspect rugueux de la peau, une absence d'élasticité et un manque d'éclat.

En vue de remédier à ces manifestations, il a été proposé des compositions cosmétiques à base de certaines vitamines liposolubles ainsi qu'hydrosolubles dans la mesure où celles-ci étaient suffisamment stables.

L'acide pantothénique (vitamine B5) a également été proposé comme ayant un effet bénéfique sur la peau, bien qu'il n'y ait pas de preuves certaines d'une pénétration à travers l'épiderme.

Ces apports complémentaires de vitamines soit par voie orale, soit par voie topique ne sont généralement pas suffisamment équilibrés en les différentes vitamines de sorte que les traitements à base de compositions polyvitaminiques ne permettent pas d'améliorer l'aspect esthétique de la peau notamment du visage.

A la suite de nombreuses études réalisées dans ce domaine, on a constaté de façon tout à fait surprenante et inattendue que des résultats particulièrement favorables quant à l'aspect esthétique de la peau, pouvaient être obtenus en combinant simultanément un traitement par voie topique et par voie orale à l'aide de certains mélanges appropriés de vitamines.

Les résultats obtenus ont en effet permis de mettre en évidence que le traitement selon l'invention améliorait de façon particulièrement significative l'aspect de la peau, celle-ci étant plus lisse, plus douce, et plus souple au toucher.

Par ailleurs, on a constaté que les personnes ainsi traitées avaient dans l'ensemble un visage présentant plus d'éclat et que par ailleurs la peau avait une meilleure résistance aux agressions extérieures.

La présente invention a donc pour objet une méthode pour améliorer l'aspect esthétique de la peau, celleci consistant à appliquer chaque jour sur la peau une composition cosmétique contenant un mélange de vitamines dans un véhicule approprié pour une application topique et à administrer journalièrement par voie orale une préparation contenant également un mélange de vitamines dans un véhicule approprié pour une telle administration, ladite méthode étant poursuivie jusqu'à l'amélioration de l'aspect esthétique de la peau.

La mise en oeuvre de la méthode selon l'invention n'implique aucun ordre particulier des deux opérations à la seule condition toutefois que chacune d'elle soit effectuée journalièrement durant le traitement.

Des résultats particulièrement appréciables tant à la vue qu'au toucher sont généralement obtenus après une période de trois à cinq semaines.

Afin de ne pas perdre le bénéfice des effets obtenus, il est particulièrement recommandé de poursuivre le traitement sous forme d'un traitement d'entretien d'une période de 3 à 4 semaines au moins une fois par an et de préférence 2 à 3 fois par an.

Le mélange de vitamines de la composition cosmétique administrée par voie topique, est essentiellement constitué de vitamines E, A, B2, B5 et H.

La vitamine E ou $\alpha$-tocophérol, de préférence sous forme d'acétate d'$\alpha$-tocophérol, est généralement présente entre 0,3 et 6% en poids et de préférence entre 1,5 et 5%.

La vitamine A ou rétinol est présente entre 0,005 et 0,5% en poids et de préférence entre 0,15 et 0,35%.

La vitamine B2 ou riboflavine, sous forme de son sel de sodium du dérivé 5' phosphate, est présente entre 0,001 et 0,01% et de préférence entre 0,002 et 0,008%.

La vitamine B5 ou acide pantothénique, de préférence sous forme de D-pantothénate de calcium ou de sodium, est présente entre 0,5 et 3% et de préférence entre 1 et 2%.

La vitamine H ou biotine est présente entre 0,01 et 0,05% et de préférence entre 0,015 et 0,03%.

Ces différents pourcentages sont ici exprimés par rapport au poids total de la composition cosmétique.

Les études qui ont été réalisées ont permis de mettre en évidence l'importance de ces différentes vitamines en vue de l'effet cosmétique recherché.

La composition cosmétique peut se présenter sous diverses formes permettant une application commode

2

de la composition sur la peau notamment sur le visage.

Ainsi celle-ci peut être par exemple une crème, un lait, un sérum ou encore une émulsion du type huiledans-l'eau ou eau-dans-l'huile.

Il convient bien entendu que le véhicule de la composition n'ait aucune incidence néfaste sur la stabilité du mélange des vitamines.

Les agents émulsionnants de ces compositions sont généralement compris entre 1 et 20% et de préférence entre 2 et 12% en poids par rapport au poids total de la composition.

Parmi les différents agents émulsionnants qui peuvent être utilisés, on peut en particulier citer :
- les esters d'acides gras polyoxyéthylénés ou polyglycérolés,
- les alcools gras oxyéthylénés ou polyglycérolés comme par exemple l'alcool oléique polyoxyéthyléné à 10 moles d'oxyde d'éthylène, l'alcool stéarylique polyoxyéthyléné de 10 à 20 moles d'oxyde d'éthylène, l'alcool oléique polyglycérolé à 4 moles de glycérol et les alcools gras synthétiques en $C_9$-$C_{15}$ polyoxyéthylénés de 5 à 10 moles d'oxyde d'éthylène,
- les sulfates d'alkyle oxyéthylénés ou non, comme le lauryl sulfate de sodium, le lauryl sulfate d'ammonium, le cétyl stéaryl sulfate de sodium, le cétyl stéaryl sulfate de triéthanolamine, le lauryl sulfate de monoéthanolamine, le lauryl éther sulfate de sodium oxyéthyléné (à 2,2 moles d'oxyde d'éthylène par exemple) et le lauryl éther sulfate de monoéthanolamine oxyéthyléné (à 2,2 moles d'oxyde d'éthylène par exemple), et des savons tels que le stéarate de triéthanolamine.

On peut également mentionner le système émulsionnant constitué de lanolate de magnésium et d'alcool de lanoline et/ou de lanoline hydrogénée.

La phase eau des émulsions peut éventuellement contenir des agents gélifiants tels que par exemple l'hydroxy propyl méthylcellulose. La phase huile constituée d'au moins une huile et éventuellement d'au moins une cire, varie entre environ 8 et 50% en poids par rapport au poids total de la composition.

Parmi les produits gras constituant la phase grasse de ces compositions, on peut citer :
- les huiles hydrocarbonées comme l'huile de paraffine, l'huile de purcellin, le perhydrosqualène et les solutions de cires microcristallines dans les huiles,
- les huiles animales ou végétales comme l'huile d'amande douce, l'huile d'avocat, l'huile de calophyllum, la lanoline, l'huile de ricin, l'huile de cheval, l'huile de porc, l'huile de sésame, l'huile d'olive, l'huile de jojoba, l'huile de karité, l'huile d'hoplostéthus, l'huile de tournesol,
- les huiles minérales dont le point initial de distillation à pression atmosphérique est d'environ 250°C et le point final de l'ordre de 410°C,
- les esters saturés ou non tels que les myristates d'alkyle comme ceux d'isopropyle, de butyle ou de cétyle, le stéarate d'héxadécyle, les palmitates d'éthyle ou d'isopropyle, les triglycérides des acides octanoïque et décanoïque et le ricinoléate de cétyle.

La composition cosmétique peut également contenir d'autres vitamines que celles énumérées, notamment de la vitamine F (Acides gras essentiels), de la vitamine D, de la vitamine B6, ou encore l'acide folique ainsi que d'autres substances pour l'entretien et l'amélioration des propriétés de la peau telles que par exemple des humectants, des agents revitalisants et des dérivés protéiniques.

Selon une forme de réalisation particulière, la composition cosmétique contient entre 0,005 et 0,025% d'acide folique et de préférence entre 0,0075 et 0,015%.

Certaines des vitamines de la composition peuvent être incorporées dans des micro-dispersions du type liposomes.

Par ailleurs, la composition peut également contenir des colorants, des parfums, des filtres solaires, des anti-oxydants ainsi que des conservateurs tels que le parahydroxybenzoate de méthyle ou le parahydroxybenzoate de propyle.

Il est préférable d'appliquer la composition cosmétique en effectuant un léger massage afin de la bien faire pénétrer, cette application étant de préférence réalisée le soir de façon à bien laisser agir et pénétrer la composition pendant la nuit.

Les compositions cosmétiques suivantes se sont montrées particulièrement favorables dans la méthode pour améliorer l'aspect esthétique de la peau selon l'invention:

1) <u>Crème du type eau-dans-l'huile</u>

<u>Mélanges de vitamines:</u>

Vitamine E..................................... 1,5%

Vitamine A..................................... 0,3%

Vitamine B2..................................... 0,003%

Vitamine B5..................................... 1%


Vitamine H..................................... 0,02%

Vitamine F..................................... 2%

Acide folique................................ 0,008%


Lanolate de magnésium............................ 7%

Alcool de lanoline............................ 3%

Myristate d'isopropyle........................... 8%

Huile de tournesol............................ 30%

Vaseline...................................... 10%

Parahydroxybenzoate de méthyle.................... 0,2%

Parahydroxybenzoate de propyle.................... 0,1%

Eau q.s.p..................................... 100%

4

## 2) Emulsion du type huile-dans-l'eau

```
Mélanges de vitamines:

        Vitamine E.............................    3%

        Vitamine A.............................    0,2%

        Vitamine B2............................    0,007%

        Vitamine B5............................    2%

        Vitamine H.............................    0,03%

        Acide folique..........................    0,015%


Perhydrosqualène...............................    10%

Polyéthylèneglycol 400.........................    3%

Propylèneglycol................................    4%

Triéthanolamine................................    0,6%

Huile d'amande douce...........................    2%

Myristate d'isopropyle.........................    1%

Esters d'acides caprique et caprylique et

d'alcool gras en C₁₂-C₁₈.......................    1%

Alcool cétylique...............................    0,5%

Acide stéarique................................    3%

Mono- et di-stéarate de glycérol non auto-

émulsionnables.................................    3%

Parfum.........................................    0,1%

Conservateurs..................................    0,2%



        Eau q.s.p..............................    100%
```

Note: d'alcool gras en $C_{12}$-$C_{18}$

### 3) Lait du type huile-dans-l'eau

Mélanges de vitamines:

Vitamine E............................................. 2%

Vitamine A............................................. 0,15%

Vitamine B2............................................ 0,002%

Vitamine B5............................................ 2%

Vitamine H............................................. 0,015%


Huile de karité....................................... 3%

Huile de vaseline..................................... 8%

Stéarate de glycérol.................................. 2%

Monostéarate de sorbitan oxyéthyléné à 20 moles

d'oxyde d'éthylène (Tween 60)......................... 1%

Acide stéarique....................................... 1,4%

Triéthanolamine....................................... 0,7%

Carbopol 940 (neutralisé)............................. 0,2%

Butylhydroxyanisol/butylhydroxytoluène (50/50)...... 0,1%

Parfum................................................ 1%

Eau + conservateurs q.s.p............................. 100%


### 4) Lait du type huile-dans-l'eau

Mélange de vitamines:


Vitamine E............................................. 2,50%

Vitamine A............................................. 0,35%

Vitamine B2............................................ 0,008%

Vitamine B5............................................ 1,50%

Vitamine H............................................. 0,02%


Sesquioléate de sorbitan.............................. 2,00%

Stéarate de glycérol oxyéthyléné à 20 moles

d'oxyde d'éthylène.................................... 2,00%

Alcool cétylique...................................... 1,00%

Huile de vaseline..................................... 10,00%


6

```
Huile de jojoba.................................    2,00%

Carbopol 940...................................    0,20%

Triéthanolamine................................    0,20%

Parfum.........................................    1,00%

Parahydroxybenzoate de méthyle.................    0,30%

Eau déminéralisée stérile q.s.p................    100%
```

La préparation polyvitaminée qui est administrée par voie orale selon la méthode de l'invention, est essentiellement constituée d'un mélange des vitamines E, A, B2, B5, H, B1 et C.

Comme on peut le constater, la préparation par voie orale et la composition cosmétique se caractérisent essentiellement par la présence de certaines mêmes vitamines à savoir les vitamines A, E, B2, B5 et H.

La vitamine A est de préférence administrée par voie orale sous forme de Provitamine A (β-carotène).

La vitamine B1 ou thiamine est de préférence administrée sous sa forme de chlorhydrate ou de nitrate et la vitamine C ou acide L-ascorbique sous forme de ses sels de calcium ou de sodium ou encore sous forme de palmitate d'ascorbyle.

Les doses journalières de ces vitamines devant être administrées selon la méthode de l'invention, en une ou plusieurs prises, sont les suivantes:

```
Provitamine A ( β-carotène)...................    1,2 à 30mg

Vitamine E ...................................    3,75 à 30mg

Vitamine B2...................................    0,3 à   5mg

Vitamine B5...................................    2,5 à 15 mg

Vitamine H....................................  0,075 à 0,45mg

Vitamine B1...................................    0,3 à 2mg

Vitamine C....................................    20 à 200mg
```

La préparation par voie orale peut également contenir d'autres vitamines notamment de la vitamine D, de la vitamine PP, de la vitamine B6 (chlorhydrate de pyridoxine), ainsi que certains éléments minéraux (Se, Cu) et oligoéléments tels que par exemple du glycérophosphate de magnésium, du citrate de magnésium, du gluconate de magnésium, du phosphate ferreux, du citrate cuivrique, du gluconate cuivrique, de l'acétate de zinc, l'histidine de zinc, du gluconate de zinc, du chlorure de zinc, des sels de sélénite ainsi que de la séléniométhionine.

Ces compositions par voie orale peuvent se présenter sous différentes formes notamment sous forme de comprimés, d'une poudre, de granulés, de pilules, de capsules, de gélules ou encore sous forme d'un sirop, d'une suspension, ou d'une solution.

Parmi ces différentes formes, celles particulièrement préférées, selon la méthode de l'invention, sont les capsules et les gélules.

Selon une forme de réalisation préférée, le mélange de vitamines est associé à au moins une huile végétale alimentaire telle que par exemple l'huile de carthame, l'huile de germes de blé, l'huile d'onagre, l'huile de bourrache, l'huile de pépins de cassis, l'huile d'arachide, l'huile de soja ou à une huile animale d'origine marine riche en acides gras polyinsaturés.

Ces huiles sont particulièrement recommandées dans la mesure où elles apportent de l'acide linoléique ainsi que de l'acide γ-linolénique (Acides gras essentiels).

La préparation par voie orale peut également contenir divers ingrédients conventionnels tels que par exemple des charges, des produits édulcorants, etc...

Les préparations suivantes se sont montrées particulièrement appropriées dans la méthode pour améliorer l'aspect esthétique de la peau selon l'invention:

A - <u>Capsule molle de gélatine de 462 mg</u>

<u>Composition par capsule:</u>

| | |
|---|---|
| Huile d'arachide............................... | 103mg |
| Huile de soja hydrogénée....................... | 15,0mg |
| Huile de carthame............................. | 85mg |
| β-carotène à 20% en suspension huileuse..... | 3,45mg |
| | (0,69mg de β-carotène) |
| Vitamine E.................................... | 7,5mg |
| Vitamine B1................................... | 0,780mg |
| Vitamine B2................................... | 0,863mg |
| Vitamine B5................................... | 5,0mg |
| Vitamine H.................................... | 0,150mg |
| Vitamine C (acide ascorbique)................. | 25,0mg |
| Levure........................................ | 70,0mg |

Afin d'avoir les doses requises en vitamines on administre deux capsules de 462mg par jour.

B - <u>Capsule molle de gélatine de 463mg</u>

<u>Composition par capsule:</u>

| | |
|---|---|
| Huile de germes de blé........................ | 168mg |
| Vitamine E.................................... | 15,75mg |
| β-carotène à 20% en suspension huileuse..... | 13,8mg |
| | (2,76mg de β-carotène) |
| Vitamine B1................................... | 0,690mg |
| Vitamine B2................................... | 0,660mg |
| Vitamine B5................................... | 10mg |
| Vitamine H.................................... | 0,150mg |
| Vitamine C (acide ascorbique)................. | 50mg |
| Acide folique................................. | 0,230mg |
| Vitamine B6................................... | 1,1mg |
| Vitamine PP................................... | 7,5mg |
| Sélénium (levure sélénium à 1000mcg/g)....... | 40mg |
| | (40mcg sélénium) |

L'administration d'une capsule de 463mg par jour apporte les doses requises de vitamines.

C - capsule molle de gélatine

Composition par capsule:

| | |
|---|---|
| Huile de pépin de cassis ................... | 200mg |
| Glutathion ........................:........ | 300mg |
| Vitamine E ................................. | 15mg |
| $\beta$-carotène à 20% en suspension huileuse .... | 15mg |
| Vitamine B2 ................................ | 1mg |
| Vitamine B5 ................................ | 4mg |
| Vitamine H ................................. | 0,250mg |
| Vitamine C ................................. | 60mg |
| Gluconate de zinc .......................... | 20mg |
| Sélénométhionine .......................... | 120mg |

L'administration d'une telle capsule par jour apporte les doses requises de vitamines.

D - Suspension

| | |
|---|---|
| $\beta$-carotène à 20% en suspension huileuse.... | 1,25g |
| | (0,25g de $\beta$-carotène) |
| Vitamine B1................................ | 0,065g |
| Vitamine B2................................ | 0,105g |
| Vitamine B5................................ | 0,55g |
| Vitamine C................................ | 2,5g |
| Vitamine E................................ | 1g |
| Vitamine H................................ | 0,005g |
| Huile de pépins de cassis q.s.p............. | 100ml |

L'administration de 2ml de la suspension par jour apporte les doses requises de vitamines.

La présente invention a également pour objet un coffret, pour la mise en oeuvre de la méthode selon l'invention, celui-ci étant constitué de deux parties, la première partie étant constituée par la composition cosmétique et la deuxième partie par la préparation par voie orale, chaque partie étant présente en des quantités permettant la mise en oeuvre de la méthode pendant au moins 15 jours.

**Revendications**

1. Méthode pour améliorer l'aspect esthétique de la peau, caractérisée par le fait qu'elle consiste à appliquer chaque jour sur la peau une composition cosmétique contenant un mélange de vitamines dans un véhicule approprié pour une application topique et à administrer journalièrement par voie orale une préparation contenant également un mélange de vitamines dans un véhicule approprié pour une telle administration, ladite méthode étant poursuivie jusqu'à l'amélioration de l'aspect de la peau.

2. Méthode selon la revendication 1, caractérisée par le fait qu'elle est poursuivie pendant 3 à 5 semaines à raison d'une mise en oeuvre journalière.

3. Méthode selon les revendications 1 et 2, caractérisée par le fait que la composition cosmétique contient un mélange des vitamines E, A, B2, B5 et H.

4. Méthode selon l'une quelconque des revendications 1 à 3, caractérisée par le fait que la composition cosmétique contient le mélange des vitamines dans les proportions en poids suivantes par rapport au poids total de la composition:
Vitamine E : entre 0,3 et 6% et de préférence entre 1,5 et 5%
Vitamine A : entre 0,005 et 0,5% et de préférence entre 0,15 et 0,35%
Vitamine B2: entre 0,001 et 0,01% et de préférence entre 0,002 et 0,008%.
Vitamine B5: entre 0,5 et 3% et de préférence entre 1 et 2%
Vitamine H : entre 0,01 et 0,05% et de préférence entre 0,015 et 0,03%

5. Méthode selon l'une quelconque des revendications précédentes, caractérisée par le fait que la composition cosmétique contient en outre de la vitamine F, de la vitamine D, de la vitamine B6 et/ou de l'acide folique.

6. Méthode selon la revendication 5, caractérisée par le fait que la composition cosmétique contient de 0,005 à 0,025% en poids d'acide folique et de préférence de 0,0075 à 0,015%.

7. Méthode selon l'une quelconque des revendications précédentes, caractérisée par le fait que la composition cosmétique se présente sous forme d'une crème, d'un lait, d'un sérum ou d'une émulsion.

8. Méthode selon la revendication 1, caractérisée par le fait que la préparation par voie orale contient un mélange des vitamines E, A (β-carotène), B2, B5, H, B1 et C.

9. Méthode selon la revendication 1, caractérisée par le fait qu'elle consiste à administrer journalièrement par voie orale le mélange des vitamines dans les doses suivantes:

```
Vitamine E.............................    3,75 à    30mg
Provitamine A (β-carotène)............     1,2 à    30mg
Vitamine B2...........................     0,3 à     5mg
Vitamine B5...........................     2,5 à    15mg
Vitamine H............................   0,075 à  0,45mg
Vitamine B1...........................     0,3 à     2mg
Vitamine C............................      20 à   200mg
```

10. Méthode selon la revendication 8, caractérisée par le fait que la préparation par voie orale contient en outre de la vitamine D et/ou de la vitamine PP et/ou de la vitamine B6 ainsi que des éléments minéraux et/ou oligoéléments.

11. Méthode selon l'une quelconque des revendications 1 à 3 et 8 à 11, caractérisée par le fait que la préparation par voie orale se présente sous forme de capsules ou de gélules.

12. Coffret pour la mise en oeuvre de la méthode selon l'une quelconque des revendications 1 à 11, caractérisé par le fait qu'il se présente sous forme d'un conditionnement en deux parties, la première partie étant constituée par la composition cosmétique et la deuxième partie par la préparation par voie orale, chaque partie étant présente en des quantités permettant la mise en oeuvre de la méthode pendant au moins 15 jours.

**Patentansprüche**

1. Verfahren zum Verbessern des ästhetischen Aussehens der Haut, dadurch gekennzeichnet, daß man täglich auf die Haut eine kosmetische Zubereitung aufbringt, die ein Gemisch von Vitaminen in einer geeigneten Trägersubstanz für die topische Aufbringung enthält, und man täglich oral eine Zubereitung verabreicht, die gleichfalls ein Gemisch von Vitaminen in einer geeigneten Trägersubstanz für eine solche Verabreichung enthält, wobei das Verfahren bis zur Verbesserung des Aussehens der Haut fortgesetzt wird.

2. Verfahren nach Anspruch 1, dadurch gekennzeichnet, daß man es während 3 bis 5 Wochen bei täglicher Anwendung fortsetzt.

3. Verfahren nach den Ansprüchen 1 und 2, dadurch gekennzeichnet, daß die kosmetische Zubereitung ein Gemisch der Vitamine E, A, B2, B5 und H enthält.

4. Verfahren nach einem der Ansprüche 1 bis 3, dadurch gekennzeichnet, daß die kosmetische Zubereitung das Vitamingemisch in folgenden Gewichtsverhältnissen, bezogen auf das Gesamtgewicht der Zubereitung, enthält:

| | |
|---|---|
| Vitamin E: | zwischen 0,3 % und 6 %, vorzugsweise zwischen 1,5 % und 5 %; |
| Vitamin A: | zwischen 0,005 % und 0,5 %, vorzugsweise zwischen 0,15 % und 0,35 %; |
| Vitamin B2: | zwischen 0,001 % und 0,01 %, vorzugsweise zwischen 0,002 % und 0,008 %; |
| Vitamin B5: | zwischen 0,5 % und 3 %, vorzugsweise zwischen 1 % und 2 %; |
| Vitamin H: | zwischen 0,01 % und 0,05 %, vorzugsweise zwischen 0,015 % und 0,03 %. |

5. Verfahren nach einem der vorhergehenden Ansprüche, dadurch gekennzeichnet, daß die kosmetische Zubereitung außerdem Vitamin F, Vitamin D, Vitamin B6 und/oder Folsäure enthält.

6. Verfahren nach Anspruch 5, dadurch gekennzeichnet, daß die kosmetische Zubereitung 0,005 Gew.-% bis 0,025 Gew.-% Folsäure, vorzugsweise 0,0075 Gew.-% bis 0,015 Gew.-% Folsäure, enthält.

7. Verfahren nach einem der vorhergehenden Ansprüche, dadurch gekennzeichnet, daß die kosmetische Zubereitung in Form einer Creme, einer Milch, eines Serums oder einer Emulsion vorliegt.

8. Verfahren nach Anspruch 1, dadurch gekennzeichnet, daß die orale Zubereitung ein Gemisch der Vitamine E, A (β-Karotin), B2, B5, H, B1 und C enthält.

9. Verfahren nach Anspruch 1, dadurch gekennzeichnet, daß man das Gemisch der Vitamine täglich oral in folgenden Dosen verabreicht:

```
Vitamin E                3,75 bis 30    mg
Provitamin A (ß-Karotin) 1,2  bis 30    mg
Vitamin B2               0,3  bis 5     mg
Vitamin B5               2,5  bis 15    mg
Vitamin H                0,075 bis 0,45 mg
Vitamin B1               0,3  bis 2     mg
Vitamin C                20   bis 200   mg
```

10. Verfahren nach Anspruch 8, dadurch gekennzeichnet, daß die orale Zubereitung außerdem Vitamin D und/oder Vitamin PP und/oder Vitamin B6 ebenso wie mineralische Elemente und/oder Oligoelemente enthält.

11. Verfahren nach einem der Ansprüche 1 bis 3 und 8 bis 11, dadurch gekennzeichnet, daß die Zubereitung zur oralen Verabreichung in Form von Kapseln oder Gelatinekapseln vorliegt.

12. Kit zur Durchführung des Verfahrens nach einem der Ansprüche 1 bis 11, dadurch gekennzeichnet, daß es in Form einer zweiteiligen Konditionierung vorliegt, wobei der erste Teil aus der kosmetischen Zubereitung und der zweite Teil aus der oral zu verabreichenden Zubereitung besteht, wobei jeder Teil in Mengen vorliegt, welche die Durchführung des Verfahrens während mindestens 15 Tagen erlauben.

## Claims

1. Method for improving the attractiveness of the skin's appearance, characterised in that it consist in applying to the skin, every day, a cosmetic composition containing a mixture of vitamins in a vehicle suitable for topical application, and in administering orally, every day, a preparation also containing a mixture of vitamins in a vehicle suitable for such an administration, implementation of the said method being continued until the appearance of the skin hat improved.

2. Method according to Claim 1, characterised in that its implementation is continued for 3 to 5 weeks at the rate of one implementation every day.

3. Method according to Claims 1 and 2, characterised in that the cosmetic composition contains a mixture of vitamins E, A, B2, B5 and H.

4. Method according to any one of Claims 1 to 3, characterised in that the cosmetic composition contains the mixture of vitamins in the following proportions by weight relative to the total weight of the composition:
   Vitamin E : between 0.3 and 6%, and preferably between 1.5 and 5%
   Vitamin A : between 0.005 and 0.5%, and preferably between 0.15 and 0.35%
   Vitamin B2 : between 0.001 and 0.01%, and preferably between 0.002 and 0.008%.
   Vitamin B : between 0.5 and 3%, and preferably between 1 and 2%
   Vitamin H : between 0.01 and 0.05%, and preferably between 0.015 and 0.03%.

5. Method according to any one of the preceding claims, characterised in that the cosmetic composition contains, in addition, vitamin F, vitamin D, vitamin B6 and/or folic acid.

6. Method according to Claim 5, characterised in that the cosmetic composition contains from 0.005 to 0.025% by weight of folic acid, and preferably from 0.0075 to 0.015%.

7. Method according to any one of the preceding claims, characterised in that the cosmetic composition takes the form of a cream, a milk, a serum or an emulsion.

8. Method according to Claim 1, characterised in that the preparation for oral administration contains a mixture of vitamins E, A ($\beta$-carotene), B2, B5, H, B1 and C.

9. Method according to Claim 1, characterised in that it consists in administering orally, every day, the mixture of vitamins in the following doses:

```
Vitamin E ...................... 3.75  to    30mg
Provitamin A (β-carotene)...... 1.2   to    30mg
Vitamin B₂ .................... 0.3   to     5mg
Vitamin B₅ .................... 2.5   to    15mg
Vitamin H ..................... 0.075 to  0.45mg
Vitamin B₁ .................... 0.3   to     2mg
Vitamin C ..................... 20    to   200mg
```

10. Method according to Claim 8, characterised in that the preparation for oral administration contains, in addition, vitamin D and/or vitamin PP and/or vitamin B6, at well at mineral elements and/or trace elements.

11. Method according to any one of Claims 1 to 3 and 8 to 11, characterised in that the preparation for oral administration takes the form of capsules including hard gelatin capsules.

12. Kit for carrying out the method according to any one of Claims 1 to 11, characterised in that it takes the form of a two-part packaging, the first part consisting of the cosmetic composition and the second part consisting of the preparation for oral administration, each part being present in amounts which enable the method to be carried out for at least 15 days.